# EUROPEAN PATENT APPLICATION

(11) **EP 1 018 328 A2**
(43) Date of publication of application: **12.07.2000**
(21) Application number: 99310478.5
(22) Date of filing: 23.12.1999
(51) Int. Cl.: A61F 13/53

(54) **A disposable absorbent article**

(30) Priority: 04.01.1999 BR 9900001
(71) Applicant: Johnson & Johnson Industria e Comercio Ltda., 05501-030 Sao Paulo, SP (BR)
(72) Inventor: Costa, Rogerio, Lorena-SP (BR)
(74) Representative: Mercer, Christopher Paul

(57) **Abstract**

The present invention relates to a disposable absorbent article (10), more specifically to a disposable absorbent article comprised of three or more longitudinal absorbing panels (40,40',40"), separated by channels and capable of generally reciprocally pivoting, thus providing a product that can be dynamically adapted to the user's body and provided with fast absorption of body fluids released thereon.

## Description

The present invention relates to a disposable absorbent article, more specifically a disposable absorbent article comprising three or more longitudinal panels separated by channels and capable of a generally reciprocal pivoting movement, thus providing a product dynamically adaptable to the users body and able to quickly absorb the body fluids released on it.

Disposable absorbent articles are consumption articles widely known in the state of the art. Typical examples are diapers for children and incontinent adults, women's napkins, bandages for wounds, breast protectors for mothers in the milk-feeding phase, sweat absorbers, and the like.

In the following, for the sake of disclosure and only for exemplification reasons, women's intimate napkins (eventually referred to as "women's napkins" or even merely as "napkins"), the object of the preferred embodiment of the present invention, are disclosed and described without excluding the protection of any other type of disposable absorbent article the features of which are inserted in the scope of the invention and claimed in the end of this specification.

Also in the text below there are references to the "top layer" and the "coating layer" which are comprised in absorbent articles known in the state of the art. In general, the known absorbent articles comprise an absorbing core sandwiched between two so-called top layer and coating layer.

The top layer, which is usually flexible and fluid permeable, is designed to contact the user's body, and aims at allowing the flow of the body fluid towards the absorbing core. In a quite common way, it is a non-woven layer or perforated plastic film.

The coating layer is impervious or resistant to the flow of fluids and is designed to prevent the fluid from leaking from the absorbing core which could otherwise contaminate and dirty the user's clothes. In a quite common way, it is a thin non-perforated plastic layer.

The top layer and the coating layer can be single or composed of more than one material having one or more coats, and typically its dimensions are a little higher than those of the absorbing core between them, so that they are joined (glued, adhered, sealed, welded, sewn, or by any another means known in the state of the art) to one another along its perimeter.

Also, in the text below mention is made to "absorbing core", another element known by those skilled in the art. In a very common way, it is comprised of cellulose fibers, paper coats, polymeric fibers, superabsorbing materials (for example, acrylate based particles or fibers which produce gel when in contact with humidity), turf moss, combinations of those materials with one another, and the like. Its purpose is to absorb and retain the body exudates, for example blood, urine, milk, sweat, etc.

In the text below, mention is also made to "side edges" (or simply "sides") and "longitudinal ends" (or simply "ends"). They are references to the peripheral parts of an absorbent article, where "side edges" are the edges parallel to the length of the absorbent and "longitudinal ends" are the tips or ends transversal to the length of the absorbent.

It should also be understood in the text below that "upper face" of the absorbent refers to the face facing the user's body and "lower face" means the face opposing the higher one facing the users clothes.

Among several desirable aspects in the performance of a woman's napkin, the anatomical adaptation, the fast absorption of body fluids and the retention of the napkin in its place during the use are especially important. Those aspects immediately reflect on the comfort and prevention of leaks during the use.

An example of state of the art articles the aim of which is the anatomical adaptation is the hourglass shape used in women's napkins, for example shown in U.S. Patents No. 3,805,790 (Kimberly-Clark Corp.) and No. 4,758,241 (Elissa D. Papajohn). That configuration takes into account the narrow space between the legs close to the vaginal area, thus propitiating an improvement in relation to rectangular shape articles.

Still another example in the state of the art of a more anatomical shape is the provision of a protuberance in the central region of a woman's napkin, in such a way that the absorbent material is close to the site the menstrual fluid is discharged. U.S. Patent No. 5,057,096 (Francis Faglione) and patent document EP249405 (Smith & Nephew Assoc. Co. PLC) are examples thereof.

With regard to higher efficacy and/or speed in the absorption of fluids, the following can be mentioned as examples of the state of the art:
- U.S. Patent No. 3,954,107 (Colgate Palmolive) that discloses two longitudinal parallel absorbing cores provided with a channel therebetween, the function of which is to serve as a deposit of fluid and is a means for directing the fluid to the longitudinal ends of the article.
- U.S. Patent No. 3,411,504 (J. A. Glassman) that discloses an absorbing core provided with longitudinal embossed lines for faster fluid transport towards the ends.

More recently, the state of the art has disclosed attempts to combine anatomical aspects with efficacy in the fluid absorption. For example, U.S. Patent No. 5,591,148 (R. R. McFall et al.) discloses a woman's napkin comprising an absorbing core consisting of three longitudinal absorbing panels, the central panel being more prominent and supported on an element that acts as a spring to keep it at a higher position, therefore closer to the user's vagina during the use. It is a product of a complex and expensive construction, different from the one of the present invention.

Still in that sense, U.S. Patent No. 5,695,324 (D. M. Weirich) discloses a woman's napkin provided with two absorbing cores, one vertically positioned with relation to the other, the aim of which is a partial penetration in the great lips of the user's genitalia. There are no joints between any of the absorbing cores, in such a way that the displacement of on affects another one, different from the present invention.

By evaluating the state of the art related to these aspects it can be noticed that there has been a constant search for an absorbent article having a better performance as to both the anatomical requirements and the absorption efficacy.

The present invention provides an improved alternative of the state of the art through the provision of a disposable absorbent article of simple and cheap construction, propitiating a dynamic and static adaptation to the user's body, concomitantly with an optimized fluid absorption.

It is a disposable absorbent article characterized by comprising an absorbing core provided with three or more substantially independent absorbing panels in the transverse direction, longitudinally oriented and separated by channels, and joining means between said absorbing panels.

In an example of a typical embodiment of the present invention, three independent, parallelepipedical parallel panels of cellulosic fiber of wood pulp constitute the absorbing core of a woman's napkin. The bases of the three absorbing panels are affixed to the coating layer, and a permeable top layer wraps the remaining part of its surfaces. The top layer is sealed to the coating layer between the bases of the adjacent absorbing panels, along a longitudinal line or band. This sealing generates a joining means that allows for the pivoting of a panel in relation to another adjacent panel. As in general the external faces of the absorbing panels (except for the bases) are wrapped by the top layer, and said same top layer is sealed to the coating layer in the space existing between each panel, a slot or channel is generated between every two adjacent panels.

When put in use, the two side absorbing panels can pivot in relation to the central absorbing panel, being laterally conformed to the geometry of the space existing between the user's legs. By receiving the discharge of body fluid, the channels allow the absorption along the whole extension of the higher and side faces of the adjacent absorbing panels, besides serving as a reservoir and longitudinal directing means for the fluid towards the napkin ends.

Some important aspects of the present invention are:
- both static and dynamic adaptation of the article to the user's body in view of the ability to move from one absorbing panel to the another, either in the time its is put into place or when the user moves, for example, by walking, seating, crossing her legs, etc;
- effective absorption, in view of the higher available surface area for receiving the fluid (the walls of the channels are surfaces that can allow the fluid to flow towards the absorbing core);
- effective leak prevention, for the channels between the absorbing panels store and direct the released fluid longitudinally, mainly in the case of abundant discharges;
- effective retention of the napkin in its place during the use, since the tensions due to the body/clothes/absorbent article interaction are substantially absorbed and/or dissipated by the movement of an absorbing panel in relation to another one.

As mentioned above, the absorbing core of the woman's napkin of the invention comprises absorbing panels which are "substantially independent in the transverse direction", wherein this expression denotes a sufficient physical separation in the direction transversal to the length, mainly in the central third of the article, for allowing one panel to pivot in relation to another one. A continuous absorbing core, contrary to that of the present invention, does not allow such movement, for the intrinsic flexibility of a continuous length of absorbent material does not allow for the pivoting in the sense of this invention. Also, it is not considered as making it possible to pivot an absorbing panel with longitudinal embossed lines (that is, resulting from a strong compression on the absorbent material), that even allow a certain degree of arching of the absorbing core applied thereto, but not enough to allow for the pivoting of an absorbing region in relation to another one. However, the present invention can encompass embodiments wherein embossed lines are used and they are perforated, segmented, partially disrupted or otherwise submitted to a mechanical or chemical treatment that can eliminate or minimize the intrinsic rigidity, thus allowing an absorbing region to pivot in relation to an adjacent one. The present invention also encompasses adjacent absorbing panels which, despite being provided with a common absorbing and/or structural base, can pivot in relation to another one.

Within the scope of the invention, the absorbing core comprises three or more absorbing panels. Advantageously, an odd number of absorbing panels is used, thus defining a central panel that stays closer to the great lips of the vulva and tends to be inserted therebetween, thus diminishing the possibility of leak. Preferably, the woman's napkin of the invention comprises three to five absorbing panels, and still more preferentially only three.

The absorbing panels of the absorbing core of the invention are "longitudinally oriented", that is, generally they follow the orientation of an imaginary longitudinal axis parallel to the length direction of the absorbent article. Preferably, said absorbing panels are substantially parallel to said imaginary longitudinal axis, but configurations wherein one or more absorbing panels show some inclination, for example, between 10 and 40 degrees with relation to the longitudinal, are not excluded. Still preferably, the absorbing panels are parallel to one another.

The absorbing panels of the absorbing core of the present invention are "separated by channels", that is, there is a substantial physical discontinuity of a panel in relation to another adjacent one, and the space between a panel and another one is deemed to be a channel which can have a varying geometry (mainly with respect to the cross-section area) during the use of the napkin, when a panel is moved in relation to the others, typically pivoting like two halves of a hinge. Any other geometry, volume or orientation of said channels are not excluded.

The absorbing panels of the absorbing core of the present invention comprises "joining means" among themselves. That means that there is a means, substract or arrangement between an absorbing panel and an adjacent one that allows that allows one panel to pivot in relation to the other. As used in the invention, the pivoting of a panel or absorbent region in relation to another is the one that occurs in a similar way to the rotation of the halves of a hinge, one in relation to the other, without the occurrence of any substantial physical change (discontinuity, partial disruption, wrinkle, and the like) of the absorbent material involved. Preferably, without excluding any another alternative, the joining means is provided as a sealing joint (line, band, points, etc.) of the top layer over the coating layer, between adjacent absorbing panels. The joining means can be situated close to any region along the thickness of the absorbing panels, and is preferably situated close to the base of said panels. The joining means can have any extension, and is typically between 30% and 100% of the length of the absorbing panels. The rotation of an absorbing panel in relation to the other is preferably between about 10 degrees about 180 degrees, and more preferably between about 15 degrees and 120 degrees.

Within another alternative embodiment of the invention, the material of the joining means between adjacent absorbing panels allows the expansion, for example, flexible, elastic or corrugated material, thus allowing a higher absorption of the tensions imposed to the absorbent article of the invention. Examples of said material would be foams, corrugated plastic films, crimped non-woven material, films with elastic zones in the region of the channels, etc.

Typically in the configuration shown in the present invention is that the base of said channels placed between the absorbing panels is the joining means itself, and is preferred that the joining means is composed by the association of the top layer with the coating layer. Alternatively, the joining means is comprised only by the top layer, or only by the coating layer, or still by the association of a distinct substract of two layers with one layer, or with another one, or both, or still any other arrangement that allows an absorbing panel to pivot in relation to another adjacent panel.

The multiple absorbing panels of the absorbing core of the invention can have identical or distinct conformations among themselves. Preferably, there is a bigger central panel which is more protruding than the other ones to, as already mentioned, tend to perform a vaginal penetration and thus diminish the possibility of leaks.

Alternatively, the central absorbing panel is provided with structure which can be conformed according to what is stated in Brazilian patent application P19005475-0 (R. Costa), the contents of which is herein incorporated by reference, preferably wherein about 5 to 75% by volume of said absorbing panel is filled with spheroidal elements having a diameter in the range between 0,1 mm and 5 mm, confined in a recess of said absorbing panel or a closure distinct from the absorbing panel and inserted therein.

In another alternative way, the absorbing panels of the article of the present invention, in particular the central absorbing panel, comprise fluid pockets, for example air, according to Brazilian patent application P19301708-1 (R. Coast), the contents of which is herein incorporated by reference. As explained in said document, the air pockets transmit a feeling of a higher initial thickness to the users who feel apprehended before using a thin product to absorb their menstrual fluid.

Preferably, the cross-section of the absorbing panels retains the same area and geometry throughout its length, but alternatives with geometric changes along its length are not excluded.

The geometric shape of the cross-section of the absorbing panels of the invention can be any one, and can not be same for all the absorbing panels. For the preferred case of three absorbing panels, some advantageous examples can be cited (the sequence below refers to shapes of the cross-section of the side panel, the central panel and the other side panel, respectively):
- quadrilateral, quadrilateral, quadrilateral;
- right triangle, quadrilateral, right triangle spectral to the other side panel;
- isosceles triangle, isosceles triangle, isosceles triangle;
- circular, circular, circular;
- capital L profile, isosceles triangle, inverted capital L profile (┘).

The channels between the absorbing panels are preferably straight and have advantageously a constant minimum width close to the joining means, between about 0.5 mm and about 10 mm, preferably between about 2 mm and 6 mm. When an absorbing panel pivots in relation to the other, the cross-section geometry of the channel changes, increasing the distance between the panels in a non-parallel way (that is, the longer the radial distance from a point to the rotation axis, the longer the distance from the adjacent absorbing panel). Alternatives with curved, winding, zigzag, and the like channels along its length are within the scope of the invention.

In an alternative embodiment of the invention, one or more absorbing panels are fully wrapped by the permeable top layer, and this way they are joined (welded, glue, or by any another adequate means) on a coating layer that preferably, in this situation, also performs the function of a joining means.

Preferably, the absorbing panels are fixed directly on the coating layer, but also it is encompassed in the scope of the invention the alternative where the panels are fixed to an intermediate substract (for example, foam, one or more thin layers of absorbent material of the paper or non-woven type, plain or corrugated material to facilitate the pivoting, etc.) which in turn is attached to or associated with the coating layer.

The absorbing panels which make out the absorbing core of the invention are typically separated, in the transverse direction, by longitudinal channels that run from a longitudinal end to the opposite one, in such a way that the absorbing panels have not absorbent material in common.

In an alternative embodiment of the invention, next to the longitudinal ends, the absorbing panels are connected to one another by a transverse band of absorbent material; in other words, the channels are interrupted before reaching the longitudinal ends, without affecting substantially the pivoting capacity, that is, the dynamic conformation of the woman's napkin of the invention. In a suitable way, said transverse band of absorbent material in the longitudinal ends is between about 1 and 3 cm wide. Still within this embodiment, the central absorbing panel (or the central absorbing panels, if there are more than one), in the longitudinal ends of the channels, can alternatively be provided with transverse embossed lines that run from one channel to the next channel, in such a way to generate a trend of a better distribution of the fluid in the transverse bands of absorbent material in the longitudinal ends of the absorbent article.

In another alternative embodiment of the invention, the absorbing panels can also comprise transverse channels or embossments along their length. Preferably, only the central panel, or central panels if that is the case, are provided with transverse channels. That configuration increases the available surface area for fluid absorption and minimizes the random wrinkling of the absorbing panels (which would otherwise occur during the use of the article and could eventually favor leaks).

The women's napkins of the invention are in a especially preferred way provided with wings or borders that extend laterally from the side edges of the article - they are elements known by those skilled in the art and their purpose is to assist the attachment of the absorbent to the external face of the region between the legs in the user's panties. It is known that said wings are side prolongations of the top layer and/or coating layer, eventually comprising absorbent material.

In that conformation with side borders, an alternative of the present invention foresees that the adjacent absorbing panels to the side edges of the absorbent extend and run partially or fully inside said borders, whose upper and lower faces, like the higher layer and lower layer, are respectively provided with permeability and impermeability. The purpose of that conformation is to prevent the absorbent from leaks. Preferably, in that embodiment, about one third of the dimension of the side border adjacent to the connection with the body of the absorbent article, in the transverse direction, is filled with absorbent material.

The present invention brings about an additional benefit to women's napkins provided with side borders, that is, the dissipation of the tensions that are generated along the generally longitudinal connection between the side borders and the body of the woman's napkin. In an usual way, the side borders are provided with adhesive regions to promote its attachment to the external surface of the region between the legs in the user's panties. That attachment is subject to tensions which grow stronger as the longitudinal ends of said ends connections are closer, so that the movements of the user nullify said attachment. The arrangement of several adjacent absorbing panels according to the invention, allowing for the movement of a panel in relation to the other, attenuates or even eliminates such tensions and limits the trend "to pull" the attachment region.

In another alternative embodiment of the invention, the channels between the absorbing panels can be continuous, as if creating islands of absorbent material in the region inside the perimeter of the woman's napkin, and in this alternative those that create substantially oblong or elipsoidal pathways are preferred. That configuration keeps the independence of the absorbing panels in the transverse direction, thus allowing the pivoting of one in relation to the other, and the absorbing core is provided with auxiliary transverse channels in the distribution of the fluid.

In another alternative embodiment of the present invention, only one channel (although it is also possible with more than one channel) of spiral configuration is used. Also in this configuration the independence of the absorbing panels is retained in the transverse direction, allowing the pivoting of one in relation to the other, and the absorbing core is provided with auxiliary transverse channels in the distribution of the fluid.

The woman's napkin of the invention can comprise any other elements known in the state of the art, like deodorants, ion exchanging resins, superabsorbents, zeolites, multiple adhesive regions for attachment of the article to the user's clothes, additional layers for spreading the fluid, etc.

With regard to the localization of adhesive regions in the lower face of the absorbent article, preferably in the case where the absorbent is provided with an odd number of absorbing panels, preferably three, it is preferred that the central absorbing panel or panels is/are not provided with adhesive regions. Or, alternatively, it is preferred that only the lower face of the side absorbing panels (there are at least two of them) is provided with adhesive regions. That alternative embodiment allows the central panels to move more freely, therefore bringing about a better conformability, and higher dissipation of tensions, the result of which is a better attachment of the napkin to the user's underclothes.

The women's napkins encompassed by the invention can have any thickness, from a few millimeters in the thinnest ones to two or more centimeters in the thickest ones. Quite obnviously, the pivoting effect of the invention is more pronounced in thicker napkins, for thicker panels are less flexible than thin absorbing panels.

Practical examples of embodiments of the invention are given below, in order to better disclose the inventive aspects thereof. The figures presented below are schematic representations without showing precise measures or dimensions, given only for exemplification purposes to elucidate the inventive aspects of the present invention. Both the examples and the figures encompass any other alternative embodiment of the invention covered by the accompanying claims.

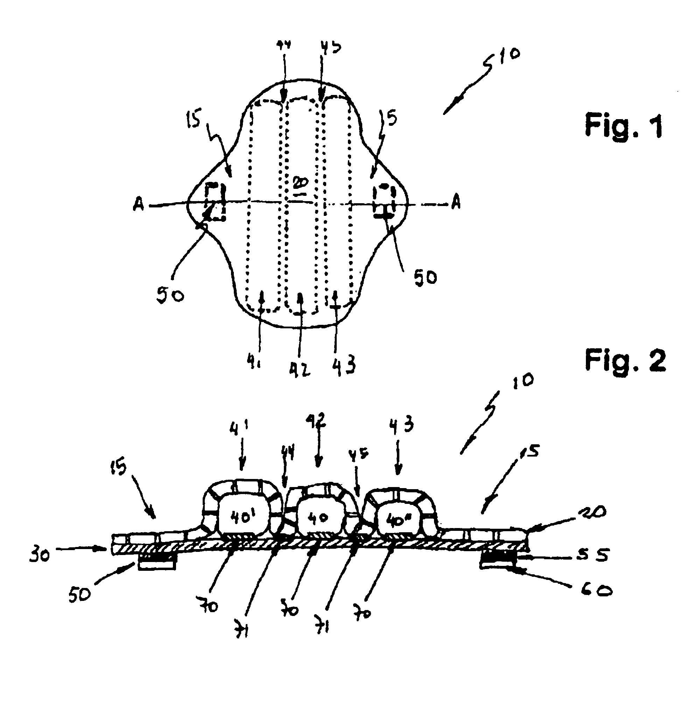

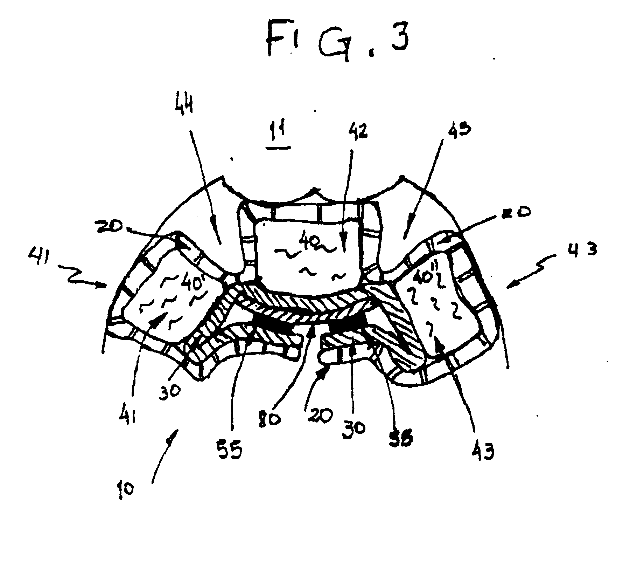

Figures 1, 2 and 3 attached hereto show the same preferred alternative embodiment of the invention, wherein figure 1 is a top view of a woman's napkin, figure 2 a cut view of figure 1 along transverse line AA, and figure 3 is another cut view, but with the absorbent article in use.

It is an external woman's napkin 10, provided with side borders 15, a top layer 20 made of a thin perforated plastic film (polyethylene, weight of 25 g/m₂, thickness of 0.04 mm, 30% open area), a coating layer 30 made of a thin impervious plastic film (polyethylene, weight of 25 g/m₂, thickness of 0.04 mm), three longitudinal parallelepipedic absorbing panels 40, 40' and 40", with a width of 2 cm, a thickness of 1 cm and a length of 150 mm, in a total 5.5 g of wood pulp. 0.5 g of an acrylate-based superabsorbent material is uniformly distributed within the wood pulp. The distance between each absorbing panel is about 0.5 cm, and each one of them forms a region 41, 42 and 43 covered by the top layer 20 in the upper and side faces, and the coating layer 30 in the base, respectively. Each absorbing panel is adhered to the coating layer 30 along a longitudinal adhesive band 70 (adhesive Findley H2465, from AtoFindley Inc., Milwakee, USA).

It is important to remember that the above mentioned components (plastic films, absorbent materials, adhesives, etc) are quite common and can be replaced by equivalents known by those skilled in the art without departing from the scope of the invention.

There is a set 50 of adhesive 55 covered by a removable protection layer 60 in the lower face of each side border.

The joining means between each absorbing panel is configured as a longitudinal band 71 resulting from the attachment between the top layer 20 top and the coating layer 30. Channels 44 and 45 (of a geometry that changes as the panels 40 pivot) are formed between the absorbing panels, as can be seen in figure 3, where the protection layer 60 was removed, and article 10 was placed on the user's genital region 11, and borders 15 have been adhered by means of adhesives 55 on the external face of the region between the legs in the panties 80.

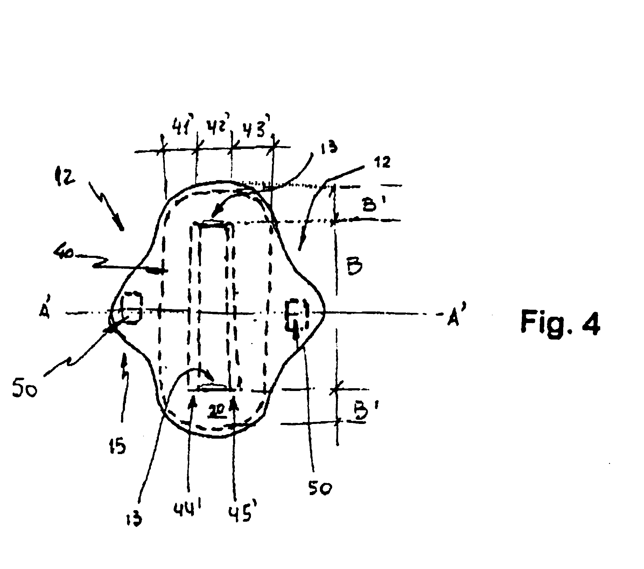

Figure 4 shows an upper view of another alternative of the invention, where a woman's napkin 12 similar to that of figure 1, with the difference that it comprises an absorbing panel 40 provided with two slits 44' e 45', both of length B, herein approximately 70% of the total length of absorbing panel 40.

Slits mean regions of physical discontinuity, where there is not substantially any absorbent material.

Although the embodiments of figure 1 and figure 4 are similar to the extent that one transverse cut along line A'A' is practically identical to that shown in figure 2, with absorbing lengths 41', 42' and 43' separated by channels 44' and 45', such channels 44' and 45' have a longitudinal extension limited to a fraction of the length of absorbent article 12, in such a way that the longitudinal ends are provided with transverse absorbent regions B'. Embossed lines 13 are present over absorbent panel 40, transversal to absorbing length 42' between channels 44' and 45', close to their ends.

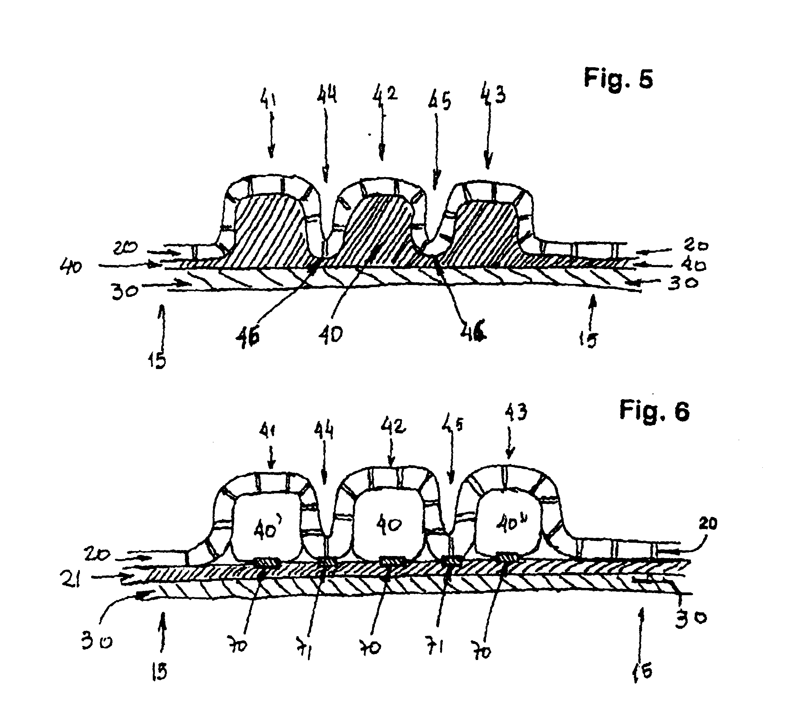

Other alternative embodiments of the invention are shown in figures 5 through 9. Figures 5, 6 and 7 are cross-section views similar to those of figure 2, showing the same numerals, except with regard to:
- figure 5: there is only one absorbing panel 40 provided with protuberances 41, 42 and 43, and bands 46 with a much lower thickness that any of those sufficient to create a continuity of the absorbent material, but without substantially jeopardizing the ability of protuberances 41, 42 and 43 to reciprocally pivot. Bands 46 act as joining means, despite the fact that they contain absorbing substract. In figure 5, absorbing panel 40 is adhered to the coating layer by means not illustrated, for example, adhesive lines.
- figure 6 - a thin sheet of paper 21 is placed between top layer 20 and coating layer 30, sufficient to provide the base of the article with some absorbing capacity, without jeopardizing the ability of absorbing panels 41, 42 and 43 to reciprocally pivot.

Regions 71, where there is an adhesion between top layer 20 and coating layer 30, between which is the sheet of paper 21, are the joining means of the article.

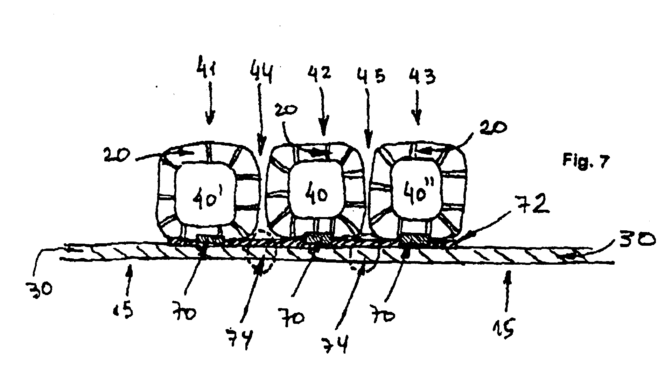

- figure 7: there are three absorbing panels 40, 40' and 40", individually wrapped by the material of top layer 20, including its bases, that are adhered to a foam layer 72 along the adhesive lines 70. Foam 72 is adhered to coating layer 30 by a means not illustrated. Regions 74 are the joining means of the article.

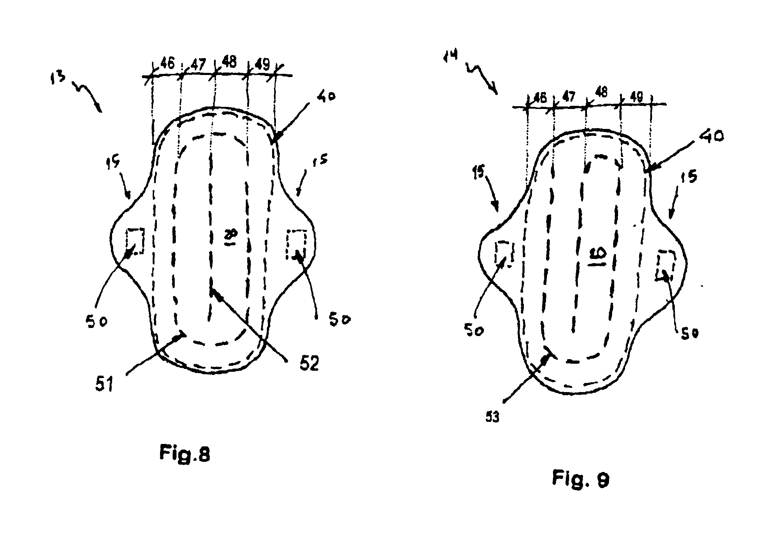

Figure 8 is a top view of an absorbent article 13 comprising only one absorbing panel 40 which is provided with two slits 51 and 52. Such configuration generates, at least in the central region of the article, regions 46, 47, 48 and 49 of absorbent material substantially independent in the transverse direction, and they are capable of substantially reciprocally pivoting.

Fig. 9 is a top view of an absorbent article 14 comprising only one absorbing panel 40 provided with only one spiral slit 53. Such configuration generates, at least in the central region of the article, regions 46, 47, 48 and 49 of absorbent material substantially independent in the transverse direction, and they are capable of substantially reciprocally pivoting.

As well illustrated in the accompanying figures, many possible changes in the embodiments of the present invention are readily noticed by those skilled in the art, always within the scope of protection as defined by the accompanying claims.

## Claims

1. A disposable absorbent article, characterized by comprising an absorbing core provided with three or more substantially independent absorbing panels in the transverse direction, longitudinally oriented and separated by channels, and comprising joining means between said absorbing panels.

2. The article of claim 1, additionally comprising borders that extend laterally.

3. The article of claim 1 or claim 2, wherein there is an odd number of said absorbing panels.

4. The article of claim 3, wherein the number of said absorbing panels is 3 or 5, preferably 3.

5. The article of claim 3 or claim 4, wherein the absorbing panel that occupies the central position is bigger and/or more protruding than the remaining absorbing panels.

6. The article of any one of claims 3 to 5 wherein the central panel is provided with a conformable structure preferably wherein 5 to 75% by volume of said central panel is filled with spheroidal elements of diameters between 0.1 mm and 5 mm, confined in a recess of said absorbing panel or a distinct closure of said absorbing panel, and inserted therein.

7. The article of any one of claims 1 to 6, wherein said absorbing panels comprise fluid pockets, preferably air.

8. The article of any one of claims 1 to 7, wherein said substantially parallel absorbing panels are substantially parallel to one another and are substantially parallel to an imaginary longitudinal axis parallel to the length direction of said absorbent article.

9. The article of any one of claims 1 to 8, wherein all said absorbing panels have substantially the same length.

10. The article of any one of claims 1 to 9, wherein said channels between absorbing panels have between 30% and 100% of the total length of said absorbing panels.

11. The article of any one of claims 1 to 10, wherein said channels between said absorbing panels are straight and preferably have a constant minimum width between 0.5 mm and 10 mm, more preferably between 2 mm and 6 mm.

12. The article of any one of claims 1 to 11, wherein said joining means are the top layer, or the coating layer, or the union of the both, along the channels between every two said absorbing panels.

13. The article of any one of claims 1 to 12, wherein said absorbing panels are parallelepipedic.

14. The article of any one of claims 1 to 13, wherein said absorbing panels additionally comprise transverse channels or transverse embossed lines.

15. The article of any one of claims 1 to 14, wherein said absorbing panels are provided with a common absorbent or structural base.

16. The article of any one of claims 1 to 15, wherein one said absorbing panel pivots in relation to said other adjacent absorbing panel between 10 degrees to 180 degrees, preferably between 15 degrees and 120 degrees.

17. The article of any one of claims 1 to 16, wherein said joining means are made of expandable or elastic material.
